# EUROPEAN PATENT SPECIFICATION

(11) **EP 1 693 379 B1**
(45) Date of publication and mention of the grant of the patent: **27.01.2010**
(21) Application number: 04802608.2
(22) Date of filing: 02.12.2004
(51) Int. Cl.: C07K 14/22, A61K 39/095, C12N 15/31, A61K 39/39, A61K 48/00, G01N 33/569

(54) **PROTEIN NMB0928 AND USE THEREOF IN PHARMACEUTICAL FORMULATIONS**
NMB0928-PROTEIN UND DESSEN VERWENDUNG IN PHARMAZEUTISCHEN FORMULIERUNGEN
PROTEINE NMB0928 ET UTILISATION DANS DES FORMULATIONS PHARMACEUTIQUES

(30) Priority: 03.12.2003 CU 28603
(43) Date of publication of application: 23.08.2006
(73) Proprietor: CENTRO DE INGENIERIA GENETICA Y BIOTECNOLOGIA, Ciudad de La Habana 10600 (CU)
(72) Inventor: PAJÓN FEYT, Rolando, La Habana 32400 (CU); GUILLÉN NIETO, Gerardo E., Ciudad de la Habana 12100 (CU); SARDIÑAS GARCÍA, Gretel Calle Jorge 18 entre, Ciudad de la Habana 10500 (CU); BETANCOURT NÚÑEZ, Lázaro, Hiram, Ciudad de la Habana 12500 (CU); CASTELLANOS SERRA, Lila, Rosa, Ciudad de la Habana 11300 (CU); PERERA NEGRÍN, Yasser, La Habana 32600 (CU); GARCÍA DÍAZ, Darién, Ciudad de La Habana 11300 (CU); NIEBLA PÉREZ, Olivia, Ciudad de la Habana 11600 (CU); CABALLERO MENÉNDEZ, Evelin Avd. 31A Edif., Ciudad de la Habana 11600 (CU); GONZÁLEZ BLANCO, Sonia Calle 184 3112, Ciudad Habana 12100 (CU)
(74) Representative: van Loon, C.J.J.
(86) International application number: PCT/CU2004/000016
(87) International publication number: WO 2005/054282

(56) References cited:
- WO-A-01/31019
- WO-A-01/37863
- WO-A-99/57280

### SEQUENCE LISTING

<110> Center for Genetic Engineering and Biotechnology
<120> PROTEIN NMB0928 AND USE THEREOF IN PHARMACEUTICAL FORMULATIONS.
<130> NMB0928
<140>
   <141>
<160> 9
<170> PatentIn Ver. 2.1
<210> 1
   <211> 32
   <212> DNA
   <213> Artificial sequence
<400> 1
   gcagatcttg gcagcaaaac cgaac 25
<210> 2
   <211> 27
   <212> DNA
   <213> Artificial sequence
<400> 2
   atggatatcc tcagctcgga atggag 26
<210> 3
   <211> 1197
   <212> DNA
   <213> Neisseria meningitidis
<400> 3
   atgccgtccg aaccgttcgg acggcataac gcaacaaaca ctttaatatc catcacacag 60
   gatgacacga tgacccatat caaacccgtc attgccgcgc tcgcactcat cgggcttgcc 120
   gcctgctccg gcagcaaaac cgaacagccc aagctcgact accaaagccg gtcgcaccgc 180
   ctgatcaaac ttgaagtccc acctgatttg aacaaccccg accaaggcaa cctctaccgc 240
   ctgcctgccg gttcgggcgc cgtccgcgcc agcgatttgg aaaaacgccg cacacccgcc 300
   gtccaacagc ctgccgatgc cgaagtattg aaaagcgtca aaggtgtccg cctcgagcgc 360
   gacggcagcc aacgctggct cgttgtcgac ggcaagtctc ctgccgaaat ctggccgctc 420
   ctgaaagcct tttggcagga aaacggcttc gacatcaaat ccgaagaacc cgccatcgga 480
   caaatggaaa ccgagtgggc ggaaaaccgc gccaaaatcc cccaagacag cttgcgccgc 540
   ctcttcgaca aagtcggctt gggcggcatc tactccaccg gcgagcgcga caaattcatc 600
   gtccgtatcg aacagggcaa aaacggcgtt tccgacatct tcttcgccca caaagccatg 660
   aaagaagtgt acggcggcaa agacaaagac acgaccgtat ggcagccctc cccgtccgat 720
   cccaacctcg aagccgcttt cctgacgcgc tttatgcaat atttgggcgt tgacggacag 780
   caggcggaaa acgcatcggc aaaaaaacct acccttcccg ccgccaacga aatggcgcgt 840
   atcgaaggca aaagcctgat tgtctttggc gactacggca gaaactggcg gcgcaccgtg 900
   ctcgccctcg accgcatcgg gctgaccgtc gtcggtcaaa acaccgaacg ccacgccttc 960
   ctggttcaaa aagccccgaa cgaaagcaat gcagttaccg aacaaaaacc cggcctgttc 1020
   aaacgcctgc tgggcaaagg caaagcggag aaacctgccg aacagccgga actgattgtc 1080
   tatgcagaac ctgtcgccaa cggctcgcgc atcgtcctgc tcaacaaaga cggcagcgca 1140
   tatgccggca aagacgcatc cgcattattg ggcaaactcc attccgaact gcgttaa    1197
<210> 4
   <211> 357
   <212> PRT
   <213> Neisseria meningitidis
<400> 4
   Cys Ser Gly Ser Lys Thr Glu Gln Pro Lys Leu Asp Tyr Gln Ser Arg
   1 5 10 15
   Ser His Arg Leu Ile Lys Leu Glu Val Pro Pro Asp Leu Asn Asn Pro
   20 25 30
   Asp Gln Gly Asn Leu Tyr Arg Leu Pro Ala Gly Ser Gly Ala Val Arg
   35 40 45
   Ala Ser Asp Leu Glu Lys Arg Arg Thr Pro Ala Val Gln Gln Pro Ala
   50 55 60
   Asp Ala Glu Val Leu Lys Ser Val Lys Gly Val Arg Leu Glu Arg Asp
   65 70 75 80
   Gly Ser Gln Arg Trp Leu Val Val Asp Gly Lys Ser Pro Ala Glu Ile
   85 90 95
   Trp Pro Leu Leu Lys Ala Phe Trp Gln Glu Asn Gly Phe Asp Ile Lys
   100 105 110
   Ser Glu Glu Pro Ala Ile Gly Gln Met Glu Thr Glu Trp Ala Glu Asn
   115 120 125
   Arg Ala Lys Ile Pro Gln Asp Ser Leu Arg Arg Leu Phe Asp Lys Val
   130 135 140
   Gly Leu Gly Gly Ile Tyr Ser Thr Gly Glu Arg Asp Lys Phe Ile Val
   145 150 155 160
   Arg Ile Glu Gln Gly Lys Asn Gly Val Ser Asp Ile Phe Phe Ala His
   165 170 175
   Lys Ala Met Lys Glu Val Tyr Gly Gly Lys Asp Lys Asp Thr Thr Val
   180 185 190
   Trp Gln Pro Ser Pro Ser Asp Pro Asn Leu Glu Ala Ala Phe Leu Thr
   195 200 205
   Arg Phe Met Gln Tyr Leu Gly Val Asp Gly Gln Gln Ala Glu Asn Ala
   210 215 220
   Ser Ala Lys Lys Pro Thr Leu Pro Ala Ala Asn Glu Met Ala Arg Ile
   225 230 235 240
   Glu Gly Lys Ser Leu Ile Val Phe Gly Asp Tyr Gly Arg Asn Trp Arg
   245 250 255
   Arg Thr Val Leu Ala Leu Asp Arg Ile Gly Leu Thr Val Val Gly Gln
   260 265 270
   Asn Thr Glu Arg His Ala Phe Leu Val Gln Lys Ala Pro Asn Glu Ser
   275 280 285
   Asn Ala Val Thr Glu Gln Lys Pro Gly Leu Phe Lys Arg Leu Leu Gly
   290                 295                 300
   Lys Gly Lys Ala Glu Lys Pro Ala Glu Gln Pro Glu Leu Ile Val Tyr
   305                 310                 315                 320
   Ala Glu Pro Val Ala Asn Gly Ser Arg Ile Val Leu Leu Asn Lys Asp
                   325                 330                 335
   Gly Ser Ala Tyr Ala Gly Lys Asp Ala Ser Ala Leu Leu Gly Lys Leu
               340                 345                 350
   His Ser Glu Leu Arg
           355
<210> 5
   <211> 1058
   <212> DNA
   <213> Neisseria meningitidis
<400> 5
   ggcagcaaaa ccgaacagcc caagctcgac taccaaagcc ggtcgcaccg cctgatcaaa 60
   cttgaagtcc cacctgattt gaacaacccc gaccaaggca acctctaccg cctgcctgcc 120
   ggttcgggcg ccgtccgcgc cagcaatttg gaaaaacgcc gcacacccac cgtccaacag 180
   cctgccgatg ccgaagtatt gaaaagcgtc aaaggtgtcc gcctcgagcg cgacggcagc 240
   caacgctggc tcgttgtcga cggcaagtct cctgccgaaa tctggccgct cctgaaagcc 300
   ttttggcagg aaaacggctt cgacatcaaa tccgaagaac ccgccatcgg acaaaaggaa 360
   accgagtggg cggaaaaccg cgccaaaatc ccccaagaca gcttgcgccg cctcttcgac 420
   aaagtcggct tgggcggcat ctactccacc ggcgagcgcg acaaattcat cgtccgtatc 480
   gaacagggca aaaacggcgt ttccgacatc ttcttcgccc acaaagccat gaaagaagtg 540
   tacggcggca aagacaaaga cacgaccgta tggcagccct ccccgtccga tcccaacctc 600
   gaagccgctt tcctgacgcg ctttatgcaa tatttgggcg ttgacggaca gcaggcggaa 660
   aacgcatcgg caaaaaaacc tacccttccc gccgccaacg aaatggcgcg tatcgaaagc 720
   aaaagcctga ttgtctttgg cgactacggc agaaactggc ggcgcaccgt gctcgccctc 780
   gaccgcatcg ggctgaccgt cgtcggtcaa aacaccgaac gccacgcctt cctggctcaa 840
   aaagccccga acgaaagcaa tgcagttacc gaacaaaaac ccggcctgtt caaacgcctg 900
   ctgggcaaag gcaaagcgga gaaacctgcc gaacagccgg aactgattgt ctatgcagaa 960
   cctgtcgcca acgggtcgcg catcgtcctg ctcaacaaag acggcagcgc atatgccggc 1020
   aaagacgcat ccgcattatt gggcaaactc cattccga                        1058
<210> 6
   <211> 1058
   <212> DNA
   <213> Neisseria meningitidis
<400> 6
   ggcagcaaaa ccgaacagcc caagctcgac taccaaagcc ggtcgcaccg cctgatcaaa 60
   cttgaagtcc cacctgattt gaacaacccc gaccaaggca acctctaccg cctgcctgcc 120
   ggttcgggcg ccgtccgcgc cagcgatttg gaaaaacgcc gcacacccac cgtccaacag 180
   cctgccgatg ccgaagtatt gaaaagcgtc aaaggtgtcc gcctcgagcg cgacggcagc 240
   caacgctggc tcgttgtcga cggcaagtct cctgccgaaa tctggccgct cctgaaagcc 300
   ttttggcagg aaaacggctt cgacatcaaa tccgaagaac ccgccatcgg acaaaaggaa 360
   accgagtggg cggaaaaccg cgccaaaatc ccccaagaca gcttgcgccg cctcttcgac 420
   aaagtcggct tgggcggcat ctactccacc ggcgagcgcg acaaattcat cgtccgtatc 480
   gaacagggca aaaacggcgt ttccgacatc ttcttcgccc acaaagccat gaaagaagtg 540
   tacggcggca aagacaaaga cacgaccgta tggcagccct ccccgtccga tcccaacctc 600
   gaagccgctt tcctgacgcg ctttatgcaa tatttgggcg ttgacggaca gcaggcggaa 660
   aacgcatcgg caaaaaaacc tacccttccc gccgccaacg aaatggcgcg tatcgaaagc 720
   aaaagcctga ttgtctttgg cgactacggc agaaactggc ggcgcaccgt gctcgccctc 780
   gaccgcatcg ggctgaccgt cgtcggtcaa aacaccgaac gccacgcctt cctggctcaa 840
   aaagccccga acgaaagcaa tgcagttacc gaacaaaaac ccggcctgtt caaacgcctg 900
   ctgggcaaag gcaaagcgga gaaacctgcc gaacagccgg aactgattgt ctatgcagaa 960
   cctgtcgcca acgcctcgcg catcgtcctg ctcaacaaag acggcagcgc atatgccggc 1020
   aaagacgcat ccgcattatt gggcaaactc cattccga                         1058
<210> 7
   <211> 1058
   <212> DNA
   <213> Neisseria *meningitidis*
<400> 7
   ggcagcaaaa ccgaacagcc caagctcgac taccaaagcc ggtcgcaccg cctgatcaaa 60
   cttgaagtcc cacctgattt gaacaacccc gaccaaggca acctctaccg cctgcctgcc 120
   ggttcgggcg ccgtccgcgc cagcaatttg gaaaaacgcc gcacacccac cgtccaacag 180
   cctgccgatg ccgaagtatt gaaaagcgtc aaaggtgtcc gcctcgagcg cgacggcagc 240
   caacgctggc tcgttgtcga cggcaagtct cctgccgaaa tctggccgct cctgaaagcc 300
   ttttggcagg aaaacggctt cgacatcaaa tccgaagaac ccgccatcgg acaaaaggaa 360
   accgagtggg cggaaaaccg cgccaaaatc ccccaagaca gcttgcgccg cctcttcgac 420
   aaagtcggct tgggcggcat ctactccacc ggcgagcgcg acaaattcat cgtccgtatc 480
   gaacagggca aaaacggcgt ttccgacatc ttcttcgccc acaaagccat gaaagaagtg 540
   tacggcggca aagacaaaga cacgaccgta tggcagccct ccccgtccga tcccaacctc 600
   gaagccgctt tcctgacgcg ctttatgcaa tatttgggcg ttgacggaca gcaggcggaa 660
   aacgcatcgg caaaaaaacc tacccttccc gccgccaacg aaatggcgcg tatcgaaagc 720
   aaaagcctga ttgtctttgg cgactacggc agaaactggc ggcgcaccgt gctcgccctc 780
   gaccgcatcg ggctgaccgt cgtcggtcaa aacaccgaac gccacgcctt cctggctcaa 840
   aaagccccga acgaaagcaa tgcagttacc gaacaaaaac ccggcctgtt caaacgcctg 900
   ctgggcaaag gcaaagcgga gaaacctgcc gaacagccgg aactgattgt ctatgcagaa 960
   cctgtcgcca acgcgtcgcg catcgtcctg ctcaacaaag acggcagcgc atatgccggc 1020
   aaagacgcat ccgcattatt gggcaaactc cattccga                         1058
<210> 8
   <211> 29
   <212> DNA
   <213> Synthetic oligonucleotide 1573
<400> 8
   ttccatggta gataaaagaa tggctttag 29
<210> 9
   <211> 27
   <212> DNA
   <213> Synthetic oligonucleotide 6795
<400> 9
   aactgcaggc ttgtaaaccg ttttgtg 27

## Description

The present invention is related to field of medicine, particularly to the development of new vaccine formulations of preventive or therapeutic application, that allow an increase in the quality of immune response against vaccine antigens of diseases from different sources.

*Neisseria meningitidis,* a Gram-negative diplococcus who's only know host is man, is the causal agent of meningococcal meningitis. Usually this bacterium is found in asymptomatic carriers among the normal population, being this niche the most common source for its microbiological isolation.

On world basis, small children less than two years of age are the more susceptible population for contracting meningococcal meningitis, however, young adults and normal adult population may also be affected.

Untreated meningococcal disease has a fatal course for most affected individuals, and vaccination could prevent this situation, by halting the events as early as at the bacterial colonization phase.

Several strategies have been developed with the aim of obtaining a vaccine able to fulfill the needed requirements in order to induce protection against this disease in general population. For this purpose capsular antigens have been taken into account, since their immunological specificity has allowed the classification into serogroups of this microorganism. Five of these serogroups have been defined as responsible of most of the clinical cases of meningococcal disease all around the world. Serogroup A is the principal cause of epidemics in sub-Saharan Africa. Serogroups B and C are associated, in most cases, to the occurrences in developed nations. Serogroups Y and W135 are common in most of the recurrent cases of the disease, and they are prevalent in some areas of USA, with a marked increase in last few years. From this data it is obvious the reason of the use, study, and evaluation of capsular polysaccharides as vaccine candidates. A tetravalent vaccine, based on capsular polysaccharides, conferring protection against serogroups A, C, Y, and W-135 has been licensed in Unite States. Elicited antibodies after vaccination are serogroup-specific (Rosenstein N. et al. 2001. Menningococcal disease. N. Engl. J. Med, 344, 1378-1388).

Serogroup B, which is different from the rest, continues to be a significant cause of endemic and epidemic meningococcal disease, and thi is mainly due to the complete lack of efficient vaccines against it. It has been noted that B capsular polysaccharide is poorly immunogenic, plus the existence of the theoretical risk for a vaccine based on this compound to induce immuno-tolerance and autoimmunity because of its structural similarity to oligosaccharide chains that are present in human neural fetal structures. (Finne J. et al. 1987. An IgG monoclonal antibody to group B meningococci cross-reacts with developmentally regulated polysialic acid units of glycoproteins in neural and extraneural tisues. J. Immunol, 138: 4402-4407). Therefore, the development of vaccines against serogroups B is concentrated in the use of sub-capsular antigens.

### Outer membrane proteins and vesicle vaccines

Initial attempts, in the 70s, to produce vaccines based on outer membrane proteins were based on the LPS depletion of outer membrane protein preparations by detergent (Frasch CE and Robbins JD. 1978. Protection against group B meningococcal disease. III. Immunogenicity of serotype 2 vaccines and specificity of protection in a guinea pig model. J Exp Med 147(3):629-44). The outer membrane proteins, OMPs, were then precipitated to produce aggregates suspended in sodium chloride. Despite promising results in animal studies, these vaccines failed to induce bactericidal antibody in either adults or children (Zollinger WD, et al. 1978. Safety and immunogenicity of a Neisseria meningitidis type 2 protein vaccine in animals and humans. J. Infect. Dis. 137(6):728-39), The poor performance of these vaccines was largely attributed to the loss of tertiary structure that accompanied precipitation. The next logical step was, therefore, to produce a vaccine with proteins displayed in their native conformation in the form of vesicles of outer membrane (Zollinger WD, et al. 1979. complex of meningococcal group B polysaccharide and type 2 outer membrane protein immunogenic in man. J. Clin. Invest. 63(5):836-48, Wang LY and Frasch CE. 1984. Development of a Neisseria meningitidis group B serotype 2b protein vaccine and evaluation in a mouse model. Infect Immun. 46(2):408-14136).

These outer membrane vesicle vaccines were significantly more immunogenic than the OMP aggregates and immunogenicity was shown to be further enhanced by adsorption to the adjuvant aluminium hydroxide (Wang LY and Frasch CE. 1984. Neisseria meningitidis group B serotype 2b protein vaccine and evaluation in a mouse model. Infect Immun. 46(2):408-14136).

A number of efficacy trials have been carried out using soluble outer membrane vesicle vaccines of different formulations. The two vaccines most extensively studied were developed in the 1980s in response to outbreaks of disease in Cuba (Sierra GV et al. 1991. Vaccine against group B Neisseria meningitidis: protection trial and mass vaccination results in Cuba. NIPH Ann Dis. 14(2):195-210) and Norway (Bjune G, et al. 1991. Effect of outer membrane vesicle vaccine against group B meningococcal disease in Norway. Lancet. 338(8775):1093-6), respectively. The OMV vaccine produced by the Finlay Institute in Cuba (commercially marketed as VA-MENGOC-BC) is produced from strain B:4:P1.19,15 with serogroup C polysaccharide and a preparation of high molecular weight OMPs and is adsorbed to aluminium hydroxide (Sierra GV et al. 1991. Vaccine against group B Neisseria meningitidis: protection trial and mass vaccination results in Cuba. NIPH Ann Dis. 14(2):195-210). This vaccine contributed to the rapid decline of the epidemic in Cuba (Rodriguez AP, et al. The epidemiological impact of antimeningococcal B vaccination in Cuba. 1999. Mem Inst Oswaldo Cruz. 94(4):433-40).

The vaccine produced by the Norwegian National Institute for Public Health (NIPH) was similarly intended initially for use during a period of hyperendemic disease caused by another organism from the ET-5 clone (B:15:P1.7,16). It was also a monovalent vaccine produced from purified outer membrane vesicles adsorbed onto aluminium hydroxide (BjuneG, et al. 1991. Effect of outer membrane vesicle vaccine against group B meningococcal disease in Norway. Lancet. 338(8775):1093-6).

Outer membrane vesicle vaccines appear to effectively present outer membrane proteins in a sufficiently natural conformation to allow the generation of functional bactericidal antibodies, at least in teenagers and adults. The antibody responses generated have also been shown to increase opsonophagocytosis of meningococci. The precise formulation of the vaccines (i.e. OMP content, LPS content and the presence or absence of adjuvant) have a significant impact on immunogenicity (Lehmann AK, et al. 1991. Immunization against serogroup B meningococci. Opsonin response in vaccinees as measured by chemiluminescence.APMIS. 99(8):769-72, Gomez JA, et al. 1998. Effect of adjuvants in the isotypes and bactericidal activity of antibodies against the transferrin-binding proteins of Neisseria meningitidis. Vaccine. 16(17):1633-9, Steeghs L, et al. 1999. Immunogenicity of Outer Membrane Proteins in a Lipopolysaccharide-Deficient Mutant of Neisseria meningitidis: Influence of Adjuvants on the Immune Response. Infect Immun. 67(10):4988-93).

The antigenic profile of disease isolates also changes rapidly and a vaccine with coverage of only a limited number of selected strains is likely to become ineffective within a few years unless the vaccine composition is changed to mirror local epidemiology.

At present OMV vaccines have been used more widely than any other serogroup B vaccine and are potentially useful in the context of outbreaks of disease caused by a single PorA type.

The immunogens that generate cross-reactivity between strains have yet to be fully defined. Studies of post-vaccination sera from both Finlay Institute and NIPH vaccine trials suggested that antibodies against both PorA (P1, the class 1 serosubtype protein) and OpcA (another major OMP, formerly known as Opc) (Wedege E, et al. 1998. Immune Responses against Major Outer Membrane Antigens of Neisseria meningitidis in Vaccinees and Controls Who Contracted Meningococcal Disease during the Norwegian Serogroup B Protection Trial. Infect Immun. 66(7): 3223-31), were both important in the mediation of serum bactericidal activity (with PorA mosl immunogenic) both these antigens show marked strain to strain variability.

The prominence of PorA protein and the significant level of variability in this protein, which appears to undergo continuous variation both between and during outbreaks (Jelfs J, et al. 2000. Sequence Variation in the porA Gene of a Clone of Neisseria meningitidis during Epidemic Spread. Clin Diagn Lab Immunol. 7(3):390-5) in epitopes to which most of the bactericidal activity in post-vaccination (and post-disease) is directed enhanced concerns that protection offered by single strain (monovalent) OMV-based vaccines might be serosubtype restricted (i.e. dependent on The PorA type).

In an attempt to overcome this potential problem, an OMV vaccine was developed in The Netherlands at RIVM that contained PorA proteins from six different prevalent pathogenic isolates (Van Der Ley P and Poolman JT. 1992. Construction of a multivalent meningococcal vaccine strain based on the class 1 outer membrane protein. Infect Immun. 60(8):3156-61, Claassen I, et al. 1996. Production, characterization and control of a Neisseria meningitidis hexavalent class 1 outer membrane protein containing vesicle vaccine. Vaccine. 14(10):1001-8). In this case the vaccine vesicles were extracted from two variants of the well-characterized H44/76 strain which had been genetically engineered lo express three separate PorA proteins. *The search for a universal antigen*

It is clear that outer membrane proteins (OMP) can induce a functional immune response against serogroup B disease but that none of the vaccines so far developed are universally protective due to the great heterogeneity of the surface exposed regions of the outer membrane proteins. The modest cross-reactive immunity induced by the outer membrane vesicles (OMV) vaccines has fuelled the search for an outer membrane antigen (or group of antigens), which induces functional antibodies and which is present on all meningococcal strains. Such antigens, if they were present on all strains irrespective of serogroup, might form the basis of a truly universal meningococcal vaccine, which would eliminate the potential problem of capsular switching on pathogenic strains following polysaccharide vaccination.

Once it became apparent that the variability of the immunodominant PorA protein would limit its use as a universal vaccine, a number of the other major outer membrane proteins were considered for their vaccine potential and several of these are under further development. Those which have been considered include class 5 proteins (OpcA), NspA and iron regulated proteins (TbpA and B, FbpA, FetA). TbpB forms part of the transferrin binding complex with TbpA. Recent work suggests that TbpA has both a greater functional role in iron binding (Pintor M, et al. 1998. Analysis of TbpA and TbpB functionality in defective mutants of Neisseria meningitidis.J Med Microbiol 47(9): 757-60) and is a more effective immunogen than TbpB.

A highly conserved minor outer membrane protein has been discovered via a novel technique using combinations of outer membrane protein preparations from different meningococcal strains to immunize mice (Martin D, et al. 1997. Highly Conserved Neisseria meningitidis Surface Protein Confers Protection against Experimental Infection. J Exp Med 185 (7): 1173-83). The B cells from the mice were used to produce hybridomas which were then screened for cross-reactivity against multiple strains of meningococci. One highly cross-reactive monoclonal antibody was found to bind to a 22 kDa outer membrane protein that was designated NspA. Immunization with recombinant NspA protein was shown to induce a cross-reactive bactericidal response in mice against strains from serogroups A-C. Vaccination also protects mice against lethal meningococcal infection (Martin D, et al. 1997. Highly Conserved Neisseria meningitidis Surface Protein Confers Protection against Experimental Infection. J Exp Med 185 (7): 1173-83). Comparison of NspA sequences among genetically divergent meningococcal strains demonstrates that the protein is highly conserved (97% homology) (Cadieux N, et al. 1999. Bactericidal and Cross-Protective Activities of a Monoclonal Antibody Directed against Neisseria meningitidis NspA Outer Membrane Protein. Infect Immun 67 (9): 4955-9).

The presence of NspA was detected by ELISA on 99.2% of tested strains from serogroups A-C using anti-NspA monoclonal antibodies (Martin D, et al. 1997. Highly Conserved Neisseria meningitidis Surface Protein Confers Protection against Experimental Infection. J Exp Med 185 (7): 1173-83). These monoclonal antibodies have been shown to be bactericidal against numerous strains of meningococci and are able to reduce meningococcal bacteraemia in a mouse model (Cadieux N, et al. 1999. Bactericidal and Cross-Protective Activities of a Monoclonal Antibody Directed against Neisseria meningitidis NspA Outer Membrane Protein. Infect Immun 67 (9): 4955-9). Although this data appears to suggest that NspA is a promising vaccine candidate that is able to protect across serogroup boundaries, polyclonal anti-recombinant NspA serum from mice does not bind to the surface of around 35% of pathogenic serogroup B meningococcal strains despite the presence of the *nspA* gene in these organisms (Moe GR et al. 1999. Differences in Surface Expression of NspA among Neisseria meningitidis Group B Strains. Infect Immun 67 (11): 5664-75).

*Antigen presentation and vaccine formulation.*

Earlier work has suggested that the form in which the antigens are presented is likely to be critical. The epitopes on membrane bound proteins are often dependent on maintenance of the correct tertiary structure and this in turn is frequently dependent on the hydrophobic membrane bound domains. It has been shown that the preparations of outer membrane proteins elicit immunity in humans only when presented in vesicle form (Zollinger WD, et al. 1979. complex of meningococcal group B polysaccharide and type 2 outer membrane protein immunogenic in man. J Clin Invest 63 (5): 836-48, Zollinger WD, et al. 1978. Safety and immunogenicity of a Neisseria meningitidis type 2 protein vaccine in animals and humans. J Infect Dis 137 (6): 728-39).

Single protein vaccines have been used in the field for decades and generally exhibit good stability. If presentation in the form of vesicles is required, to allow the antigens to remain membrane bound, stability and reproducibility may be difficult to guarantee. The immunogenicity and reactogenicity of outer membrane vesicles may vary with alterations in the amount of protein and LPS removed in the purification processes. A substantial body of experience in vesicle production has accrued in OMV vaccine manufacture, however, and the currently produced vaccines are subject to thorough quality control. Construction of entirely synthetic liposome vesicles may allow further optimization and standardization of such vaccines (Christodoulides M, et al. 1998. Immunization with recombinant class 1 outer-membrane protein from Neisseria meningitidis: influence of liposomes and adjuvants on antibody avidity, recognition of native protein and the induction of a bactericidal immune response against meningococci. Microbiology 144(Pt 11):3027-37). In other words, outer membrane proteins have been presented both, in vesicles and as pure expressed proteins, and the development of antibody responses has been modest. Main efforts so far have concentrated on intramuscular injection of meningococcal vaccine, leading to the production of systemic IgG. However, in meningococcal disease where invasion of the host is via the nasal epithelium, the production of secretory IgA may also be important.

*The N. meningitidis* genome sequence

The genome sequences of MC58 (a serogroup B meningococcus) (Tettelin H, et al. 2000. complete Genome Sequence of Neisseria meningitidis Serogroup B Strain MC58. Science 287 (5459): 1809-15172) y and of Z2491 (a serogroup A strain) (Parkhill J, et al. 2000. complete DNA sequence of a serogroup A strain of Neisseria meningitidis Z2491. Nature 404 (6777):502-6173) were elucidated and published during 2000. WO 99/57280 discloses genomic sequences of *Neisseria meningitidis,* describes the presence of open reading frames therein and predicts the existence of proteins useful as antigens for Neisseria vaccines based therein. WO 01/31019 and WO 01/37863 disclose additional predicted protein sequences based on open reading frames in genomic sequences of *Neisseria meningitidis* The availability of the annotated gene sequences should have a dramatic influence on meningococcal vaccine research. While the MC58 genome sequencing was in progress, Pizza et al. began identifying the open reading frames that were predicted to encode either membrane bound, surface exposed or exported proteins. They identified 570 such ORFs, amplified them via the polymerase chain reaction and cloned them into *Escherichia coli* to allow expression of the encoded proteins as either His-tagged or glutathione S-transferase fusion proteins (Pizza M, et al. 2000. Identification of Vaccine Candidates Against Serogroup B Meningococcus by Whole-Genome Sequencing. Science 287 (5459): 1816-20). The 61% (350) of the selected ORFs were successfully expressed, those which failed to express were often those containing more than one hydrophobic trans-membrane domain (possibly excluding a number of outer membrane bound proteins). The recombinant proteins were purified and used to vaccinate mice. The immune sera were then assessed for surface binding to multiple meningococcal strains by enzyme linked immunosorbent (ELISA) assay and flow cytometry and for bactericidal activity against two strains using the serum bactericidal assay. Finally seven proteins were selected for further study on the basis of a positive response in all three assays. Trial vaccine formulations using a number of these proteins in combination with adjuvants have been shown to induce significant bactericidal tires against the homologous meningococcal strain (MC58) in mice, but none of the proteins induced SBA litres as high as an MC58 outer membrane vesicle vaccine (Giuliani MM, et al. 2000. Proceedings 12th IPNC. p. 22). On the other hand, there is some evidence that combinations of these proteins may exhibit higher immunogenicity in mice than single proteins (Santini L. et al. 2000. Proceedings 12th IPNC. p. 25). The numerous open reading frames which were excluded during this work, perhaps through failure of protein expression or modification of their immunological properties, may also have vaccine potential and require further investigation.

Vaccine components may be selected more effectively once an understanding of the contribution of individual antigens to the pathogenesis of *N. meningitidis* has been gained. The antigens themselves may make effective vaccine candidates or, alternatively, the attenuated mutants could be considered as vaccine constituents.

In this direction, the use of vaccine candidates with a high degree of sequence conservation among several species of pathogenic microorganisms, could provide a solution to the multiple diseases they might cause in the case that these candidates induce a convenient response through the action of the immune system.

The technical aim that this invention pursues is the development of vaccine formulations capable of increasing and/or broadening the induced immune response against different pathogens or against a wide range of individual pathogens variants being thes pathogens of cancer, bacteria, viral or any other origin.

### Description of the invention

In the work object of the present invention it is reported, for the first time, the use of the NMB0928 protein as a component of a vaccine formulation with therapeutic or preventive character against the meningococcal disease or any infection caused by a member of the *Neisseria* genus.

The novel character of this invention consists in the use, previously unreported, of the NMB0928 protein in formulations with new properties, able to induce a systemic and mucosal immune response of broad-spectrum protection, due to the conserved character of this protein in different isolates of *Neisseria meningitidis* and *Neisseria gonorrhoeae.*

The scope of the invention is defined by the claims.

### Brief description of drawings

**Figure 1****.** Cloning vector pM100 employed in the cloning and expression of protein NMB0928. pTrip, tryptophan promoter; N-term P64k, P-64k N-terminal fragment; T4 Terminator, Transcriptional terminator T4 phage.
**Figure 2****.** Final construction of nucleotide sequence of the gene *NMB0928* in pM100 vector.
**Figure 3****.** SDS-PAGE analysis of fractions obtained from cellular disruption; Lane 1, supernatant; Lane 2, cellular pellet.
**Figure 4****.** SDS-PAGE analysis of the solubilization process of protein NMB0928 starting from the disruption pellet: (A) lane 1, disruption pellet; lane 2, pellet after the wash with 1X TE buffer containing 3M urea; lane 3, soluble fraction resulting from the wash; (B) lane 1, supernatant of the solubilization with 1X TE buffer containing 6M urea; lane 2, solubilization pellet.
**Figure 5****.** Antibody levels (IgG) against recombinant protein NMB0928, obtained after mice immunization with the same antigen by intra-nasal or intra-peritoneal route. ELISA results are represented, which were expressed, as the inverse of the highest dilution that duplicates the value of pre-immune sera.
**Figure 6****.** Recognition by *Western blotting* of the NMB0928 protein present in *N. meningitidis* OMVs using sera from mice immunized with the recombinant protein: The arrow indicates the band corresponding to the immuno-identified NMB0928 protein.
**Figure 7****.** IgA antibody response against recombinant protein NMB0928, at mucosal level, in mice immunized with the antigen by intra-nasal route. Results are expressed as the inverse of the highest dilution that duplicates the value of pre-immune sera. (A) IgA antibody response in saliva. (B) IgA antibody response in lung washes.
**Figure 8****.** Results of homology searches between NMB0928 protein ("query") and anotated sequences in genomes from different serogroups of *Neisseria meningitidis* ("Sbjct") using the program BLAST.
**Figure 9****.** Recognition of NMB0928 protein in different strains of *N. meningitidis,* by sera elicited against the recombinant antigen. In the graphic only are shown the results obtained when using semi-purified protein by intra-peritoneal route, however a similar behavior was observed in the rest of the cases. Results are expressed as the inverse of the highest dilution that duplicates the value of pre-immune sera.
**Figure 10****.** Comparison among the sera elicited by immunization with the protein obtained by two methods, administered by intra-peritoneal route, in the passive protection experiments against meningococcal infection, in the infant rat model.
**Figure 11****:** Recognition of protein NMB0928 and a panel of un-related antigens by generated mAbs (mAbs E45-8-15, 2G23-12). P1, Class 1 protein *Neisseria meningitidis* strain B:4:P1.15; P64k, E3 subunit of pyruvate dehydrogenase from *Neisseria meningitidis;* T.T, tetanus toxoid; HBsAg, Hepatitis B surface Antigen.
**Figure 12****.** Recognition of NMB0928 protein by human convalescent sera from survivors of meningococcal disease. As negative control healthy donor sera were employed. Results are shown as the absorbance (492nm) in an ELISA type assay.
**Figure 13****.** JY1 anti-peptide titers from the sera of animals immunized with either free peptide (JY1), recombinant protein (NMB0928) or the conjugate JY1- NMB0928.

### Examples.

### Example 1

### Detection of NMB0928 protein in serogroup B Neisseria meningitidis outer membrane vesicles preparations

With the aim of studying proteins that are present in serogroup B Neisseria meningitidis (strain B:4:P1.19,15) outer membrane vesicles, a bi-dimensional electrophoresis was carried out according to a method described elsewhere (Görg A, et al. 1985. Electrophoresis 6:599-604). Subsequently an enzymatic digestion was made upon the gel extracted proteins using trypsin (Promega, Madison, WI, U.S.). Peptides generated after digestion were extracted into solution by using microcolumns (ZipTips, Millipore, MA, U.S.). For mass spectometry analysis peptides were eluted from microcolumns with acetonitrile 60%, formic acid 1% followed by an immediate application into nanotips (Protana, Denmark).

Measurements were carried out in a hybrid mass spectrometer with cuadrupole and time of flight (QTof-2^{™}, Manchester, United Kingdom), fitted with an ionization source (nanoESI). Mass spectrometry data were acquired in a w/z range of 400-2000 in 0.98 seconds and using 0.02 seconds between scannings. Data acquisition and data processing were carried out using the MassLynx program (version 3.5, Micromass). Protein identification based on mass spectrum data was carried out using the ProFound program (Zhang W and Chait BT. 2000. ProFound: an expert system for protein identification using mass spectrometric peptide mapping information. Anal Chem 72:2482-2489. http://prowl.rockefeller.edu/cgi-bin/ProFound). The search was subscribed to the genes and derived protein sequences contained in the SwissProt database (http://www.ebi.ac.uk/swissprot/) and NCBI (http://www.ncbi.nlm.nih.gov/), considering the oxidation of methionines, deamidation and carboxyamidomethylation of cysteines as possible modifications to be encountered.

Identification of proteins based on the mass spectra was carried out with the MASCOT program (Perkins DN, et al. 1999. Probability-based protein identification by searching sequence databases using mass spectrometry data. Electrophoresis 20:3551-3567. http://www.matrixscience.com/). Search parameters included cysteine modifications as well as oxidations and deamidations.

Starting from the analysis of results obtained from the identification of proteins present in preparations of outer membrane vesicles, the NMB0928 protein was selected to be evaluated as possible vaccine candidate, from which one peptide was identified by mass spectrometry.

### Example 2

### Identificación del producto del gen nmb0928 como la lipoproteina-34 de Neisseria meningitidis

For the identification of the NMB0928 protein, a sequence homology search was done in the NCBI data base employing the BLAST program (Altschul SF, et al. 1990. Basic local alignment search tool. J Mol Biol 215:403-410, http://www.ncbi.nlm.nih.gov/BLAST/). The results of this procedure indicated homology with, in addition to the corresponding protein in other serogroups of Neisseria, with the one in several microorganisms, including lipoprotein -34 codified by the *nlpB* gene from *Escherichia coli,* identified in 1991. It is demonstrated that this protein is fractionated in the outer membrane proteoliposomes (Bouvier J, Pugsley A.P and Stragier,P. 1991. A gene for new lipoprotein in the dapA-purC interval of the E. coli chromosome. J Bacteriol 173(17):5523-31)

The conservation of this protein in the genome of several microbial genus, has resulted in their inclusion as a group of orthologous proteins in a conserved domain reported by NCBI [gnI|CDD|12651, COG3317, NIpB, Uncharacterized lipoprotein (Cell envelope biogenesis, outer membrane)], which indicates a common phylogenetic ancestor for all of them.

The analysis of the neighbourhood of these genes employing the MBGD data base (Uchiyama, I. 2003. MBGD: microbial genome database for comaprative análisis. Nucleic Acids Res. 31, 58-62.), revealed a significant similarity in the gene organization, leading to the identification of the NMB0928 protein as the lipoprotein-34 (NIpB) of *Neisseria meningitidis.*

### Example 3

### Cloning and expression of the NMB0928 gene, codifying for NMB0928 protein from N. meningitidis in Escherichia coli.

In order to clone and express the NMB0928 gene, the pM-100 cloning vector was employed. This vector allows the cloning to be carried out using different restriction enzymes and the generation of high expression levels of heterologous proteins in the form of inclusion bodies in *E*. *coli.*

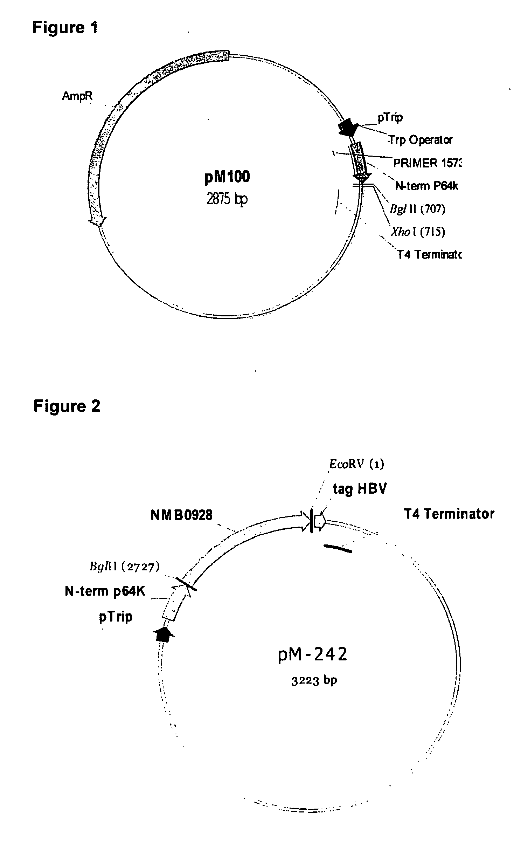

The pM-100 vector (Figure 1) have the following elements: tryptophan promoter, gene segment codifying for the 47 amino acid stabilizing sequence from Nt-fragment of P64 kDa from *N. meningitidis* strain B:4:P1.19,15, sequence of bacteriophage T4 transcriptional terminator, and the sequence of the gene that confers resistance to ampicillin as selection marker.

From the nucleotide sequence codifying for NMB0928 protein (Example 1) two primers were designed (7740 y 7741) in order to amplify the segment of this gene, without the sequence that codifies for the predicted signal peptide, from the strain B:4:P1.19,15 genomic DNA.

For the prediction of signal peptide the SignaIP World Wide Web server (http://www.cbs.dtu.dk/services/SignaIP-2.0) was employed.

After PCR amplification of the *NMB0928* gene (Randall K, et al. 1988. Science 42394:487-491) employing primers 7740 and 7741, the PCR product was digested using BgIII and EcoRV restriction enzymes, and cloned into vector previously digested pM-100 cloning vector. The final construction is showed in Figure 2, and the NMB0928 protein is expressed as a fusion protein to the Nt-segment of P64 kDa protein. Sequencing of the cloned gene NMB0928 was carried out using ALFexpress II automatic sequencer (Termo Sequenase^{™} Cy^{™} 5 Dye Terminador Kit, Amersham Biosciences) and oligonucleotides 1573 (Seq. ID. No. 8) and 6795 (Seq. ID. No. 9), that bind the sequence of the P64 stabilizer and T4 transcriptional terminator, respectively. The plasmid generated herein was designated pM-242 for its posterior use.

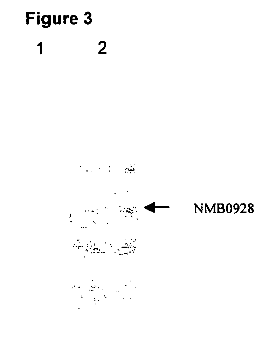

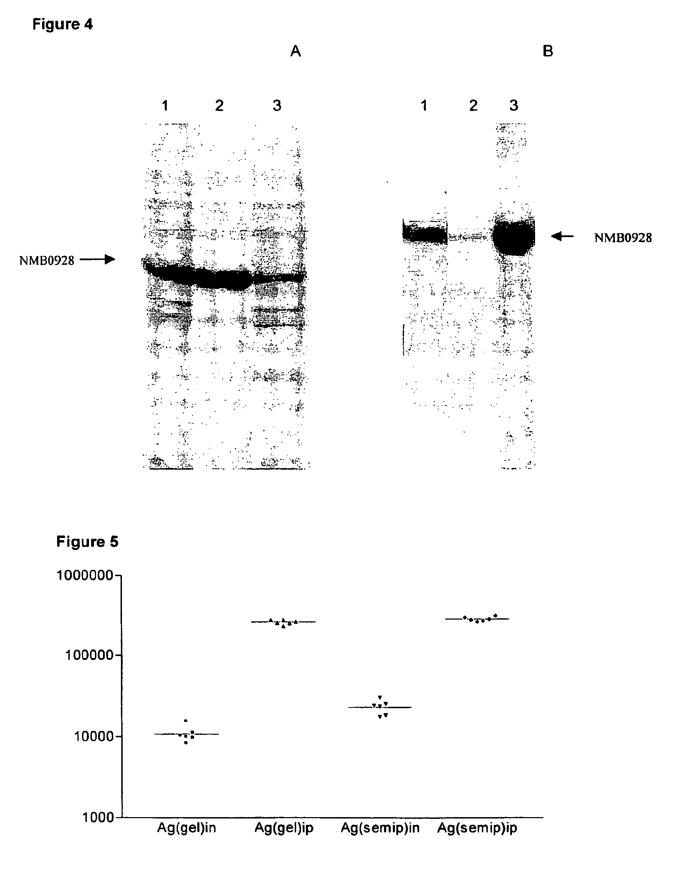

For the expression of the NMB0928 gene the GC366 *E. coli* strain was transformed by the chemical method with the pM-242 plasmid (Figure 2). The expression experiment was carried out in minimal media (M9) (Miller JH. 1972. Experiments in Molecular Genetics, Cold Spring Harbor Laboratory Press, NEW York, USA) supplemented with 1% glycerol, 1% casein hydrolisate, 0.1 mM CaCl₂, 1mM MgSO₄ and 50 ug/mL ampicillin. Bacterial cultures were incubated 12 hours at 37 °C and 250 rpm. Grown cultures were centrifugated and ultrasonic disruption of the cellular pellet was performed (IKA LABORTECHNIK). Fractions from pellet and supernatant were analyzed by SDS-PAGE (Laemmli UK. 1970. Cleavage of structural proteins during the assembly of the head of bacteriophage T4. Nature 277:680) plus stain with Coomassie Brilliant Blue R-250. The percent of expression was carried out by gel densitometry (LKB Bromma 2202 Ultrascan laser densitometer; Amersham Pharmacia Biotech, United Kingdom). The NMB0928 protein was obtained from the pellet fraction, being about the 60% of total protein content of this fraction (Figure 3). The pellet was washed with 1x TE buffer (10 mM Tris-hydroxymethyl aminomethane, 1 mM ethylendiamino tetracetic acid, pH 8) containing 2M urea, and some contaminants passed to the supernatant and the NMB0928 protein remained in the pellet (Fig 4A). Then, the pellet was solubilized with 1x TE buffer containing 6M urea, and the referred protein passed to the soluble fraction which was dialyzed against 1x TE buffer, resulting in a final preparation with 70% purity as it can be observed in Figure 4B.

### Example 4

### Evaluation of the immune response induced after immunization with NMB0928 protein by intra-peritoneal and intra-nasal routes.

To evaluate the immunogenicity of the protein NMB0928, an immunization experiment was designed and conducted in mice, where the same protein was administered by two different methods. The first consisted in to extract the band from a polyacrylamide gel (Castellanos L, et al. 1996. A procedure for protein elution from reverse-stained polyacrylamide gels applicable at the low picomole level: An alternative route to the preparation of low abundance proteins for microanalysis. Electroforesis 17: 1564-1572) and the second one was referred in Example 3, and the product was denoted as semi-purified protein.

With these preparations, female Balb/C mice (8-10 weeks-old) were immunized, once divided in 4 groups of 8 mice, each. Three immunizations were applied by intra-nasal or intra-peritoneal route, with 15 days-interval in between. The protein administered by intra-peritoneal route was emulsified with Freund's adjuvant. In Table 1 is described the composition of the groups:

**Table 1: Groups of Balb/C mice employed for immunization**

| Groups | Prot. extracted from gel | Semi-purified protein | Route |
|---|---|---|---|
| 1 | 50µg | -- | i.n |
| 2 | -- | 50µg | i.n |
| 3 | 10µg | -- | i.p |
| 4 | -- | 10µg | i.p |

The antibody titers (IgG) against the recombinant protein and the homologous protein present in the bacterium were determined by an ELISA, in serum samples taken after the third inoculation. In Figure 5, the antibody titers against the recombinant protein of individual animals are shown. Antibody levels were determined after the second inoculation, although they were higher after the third inoculation. Moreover, the immunoidentification by *Western blotting* was done, where the respective protein band was recognized. The groups immunized by intra-peritoneal route had titers significantly higher than those elicited by intra-nasal route. For the statistical analysis of the results, the non-parametric analysis of variance of Kruskal-Wallis was used, due to the non-homogeneity of the variance in the groups, according to the Bartlett's test. The Multiple comparison test of Dunn was employed to compare the means of each treatment.

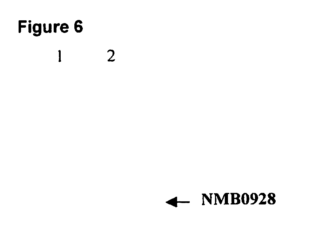

The sera obtained after the immunization with the recombinant protein recognized the natural protein present in a preparation of outer membrane protein (OMP) of strain CU385. These results are represented in Figure 6.

To analyze the mucosal response saliva samples and lung washes were evaluated.

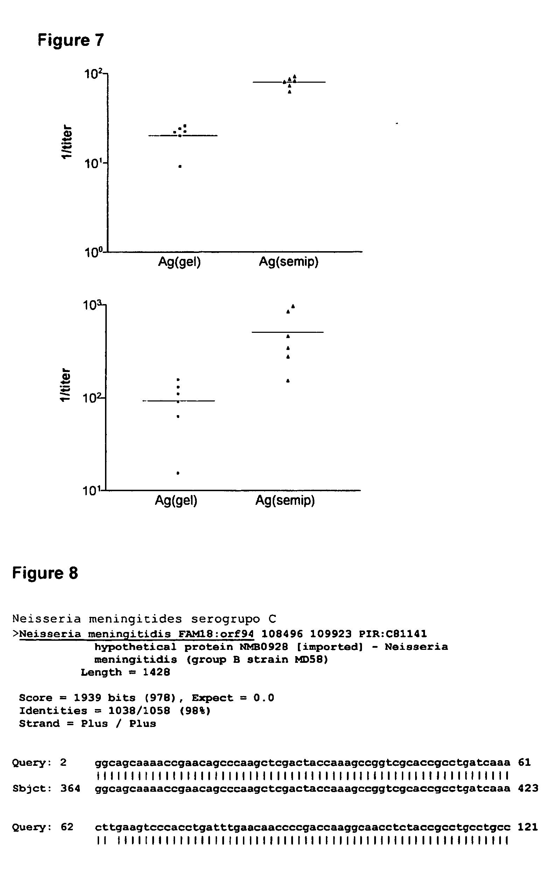

Figure 7 show only the groups immunized by intra-nasal route. An increase in the IgA titer was observed in the group that received the semi-purified protein.

### Example 5

### Characterization of the sequence of the gene codifying for protein NMB0928 in different strains of N. meningitidis.

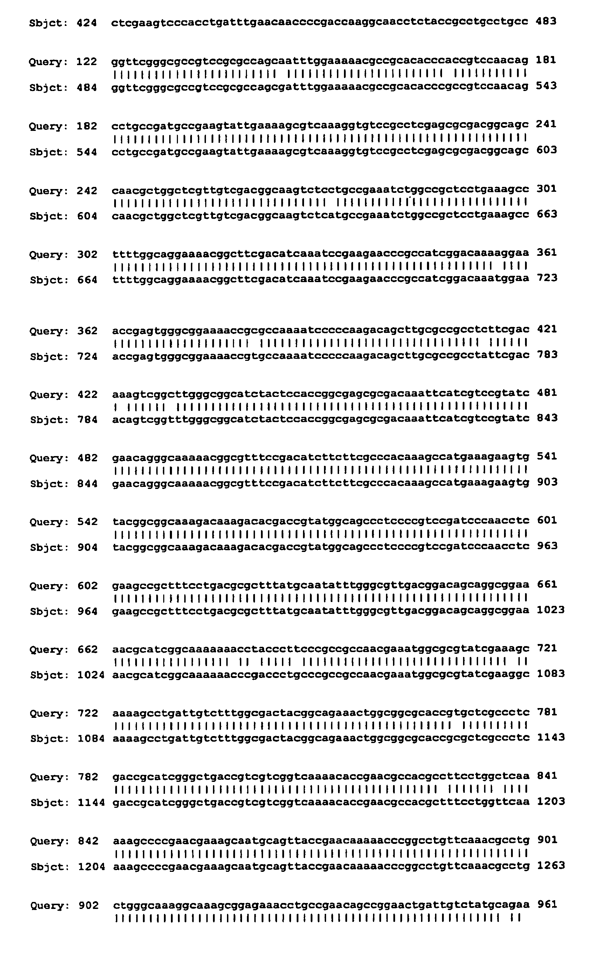

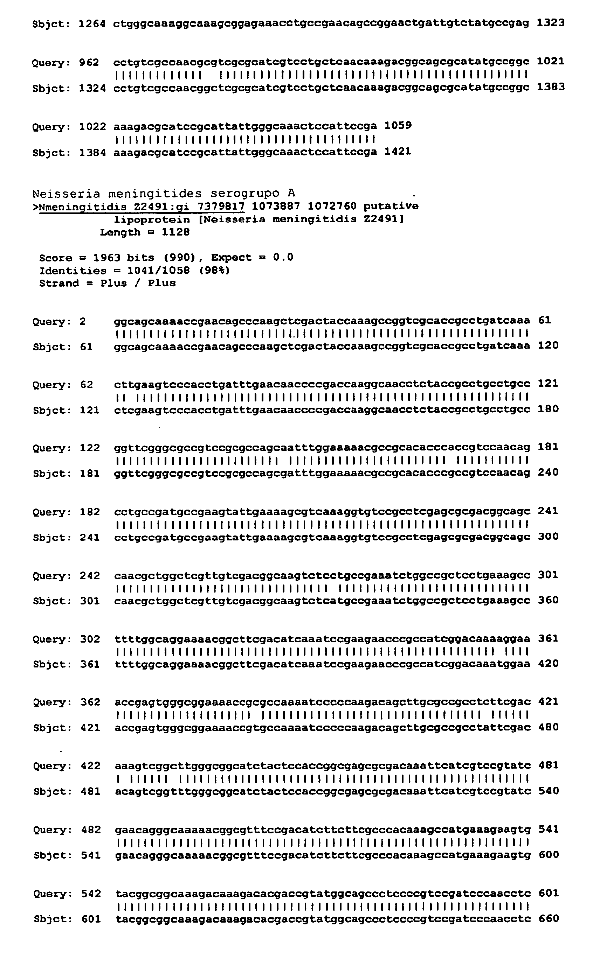

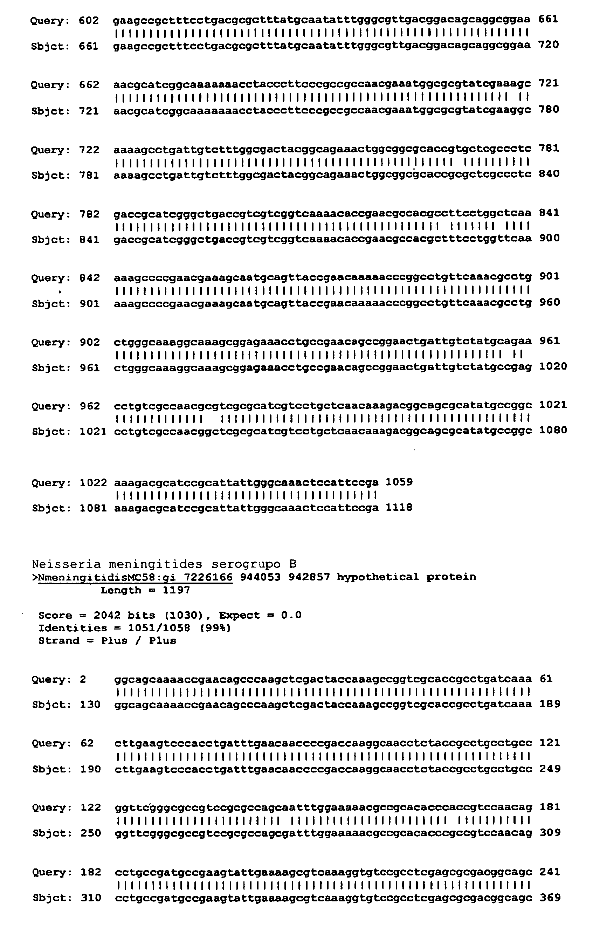

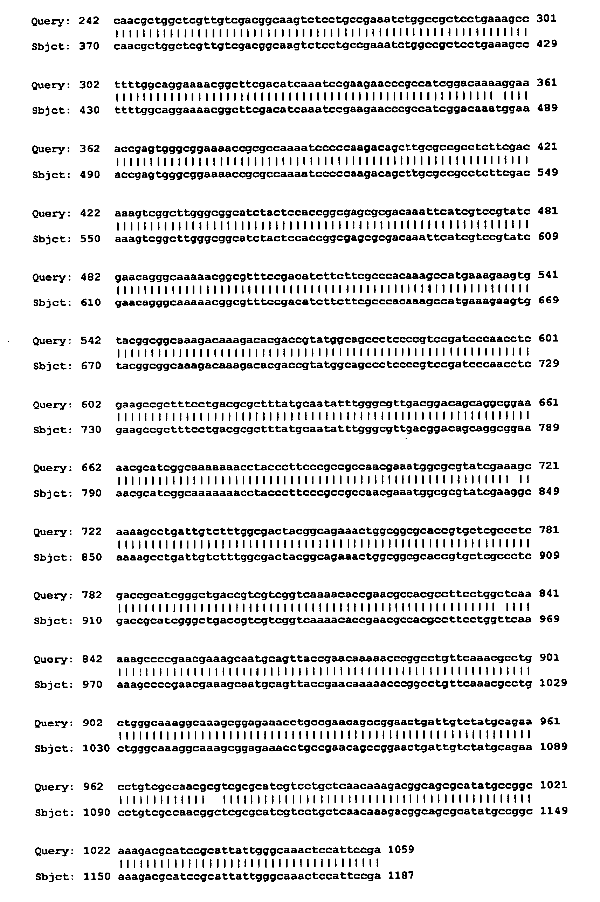

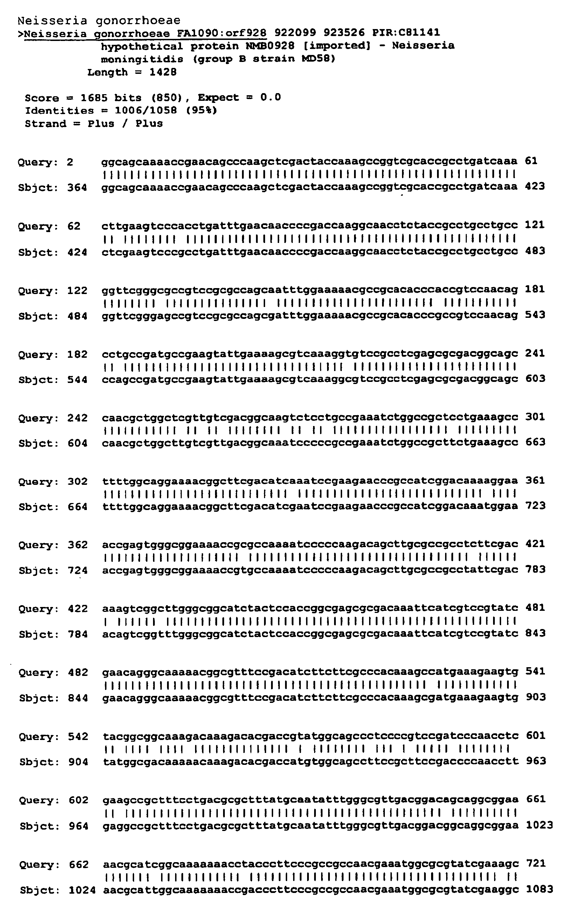

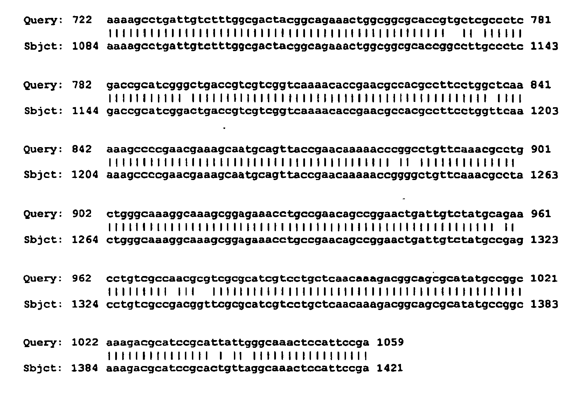

To analyze the conservation of the sequence of the gene codifying for the NMB0928 protein in the pathogenic species of the Neisseria genus a similarity search with the genomes of *Neisseria meningitidis* (serogroups A, B and C) and *Neisseria gonorrhoeae,* annotated in the NCBI data base, was done (NC 003116.1, NC 003112.1, NC 003221, NC 002946 SANGER 135720|Contig1) employing the BLAST program (Altschul SF, et al. 1990. Basic local alignment search tool. J Mol Biol 215:403-410. http://www.ncbi.nlm.nih.gov/BLAST/). Figure 8 shows the results of the sequence comparison for those sequences that produce a significant aligment in each of the analyzed genomes. Those sequences have 98% identity in serogroups A and C, 99% identity in serogroup B and 96%identity with *Neisseria gonorrhoeae,* with the sequence obtained for the gene that codicies for the NMB0928 protein (Seq. ID. No. 3). In addition, the sequence of the referred gene was determined for 3 Cuban isolates (Seq. ID. No. 5-7), which belong to serogroup B (B:4:P1.19,15) and a sequence alignment was done by using the ClustaIX program (http://www.ebi.ac.uk/clustalw/). The results of the alignment show that there is a great conservation in the nucleotide sequence of the gene NMB0928 among the analyzed strains.

The use of the protein NMB0928 as a vaccine candidate, taking into account the high degree of similarity existing among the sequences previously mentioned, would allow the generation of an effective immune response, with a broad-spectrum protection (due to the cross reactivity) against the meningococcal disease.

### Example 6

### Characterization of the immune response with broad-spectrum action induced by the immunization of Balb/C mice with the protein NMB0928.

To evaluate if the immunization with protein NMB0928 induced a response broadly cross-reactive with other strains of Neisseria, an ELISA was done. The polystyrene plates were coated with whole cells of 7 strains of Neisseria, which belong to different serotypes and serosubtypes. The plates were incubated with pooled sera obtained against the protein NMB0928, by two routes of immunization, as described in Example 4.

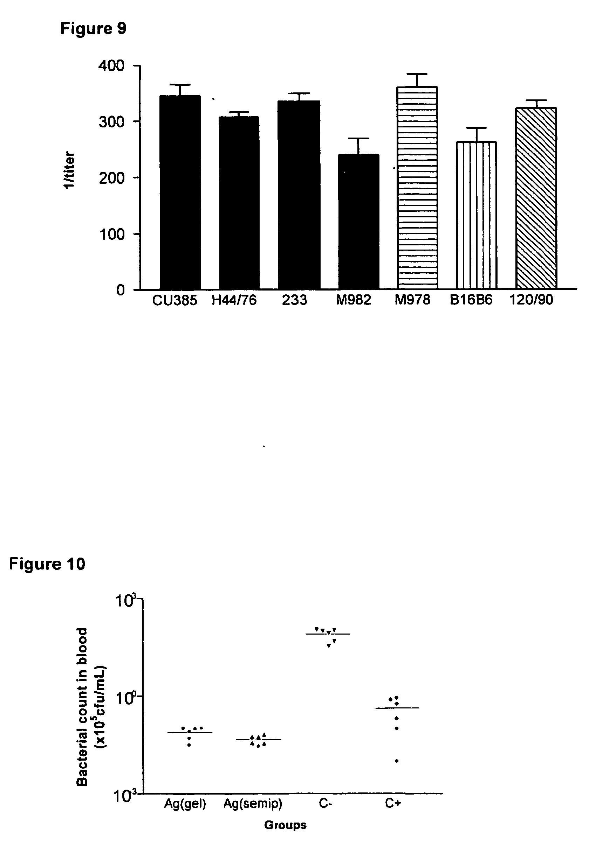

Figure 9 shows the results obtained with the sera elicited against the semi-purified protein administered by intra-peritoneal route. As it is observed, the immune sera recognized the protein present in different strains, with levels similar to the one found in the strain CU385. The rest of the sera had a comparable behavior in this assay.

### Example 7

### Protection induced by the murine sera specific for protein NMB0928, against homologous and heterologous strains, in the infant rat model

To determine the functional activity of the antisera obtained, a protection assay was conducted in the infant rat model for meningococcal infection. Twenty four rats (5-6 days old) were divided in groups of 6 rats each.

It was determined if the sera administered by intra-peritoneal route protected the rats from the infection caused by bacteria (strain CU385), inoculated by the same route one hour later. The sera of each group were pooled and diluted 1/10 (in sterile PBS) before they were inoculated in infant rats. Four hours later, the animals were sampled and viable bacteria in their blood were counted.

To interpret the results, an Analysis of Variance (Anova) was done, followed by a Dunnet's Multiple Comparison Test, where the test groups were compared with the negative control. As it is observed in Figure 10, the groups that received antisera.

A similar assay was done infecting infant rats with strains M982 and 120/90, isolated from Cuban patients, which serological classification is homologous to the strain B385. Moreover, challenge experiments were conducted with strain 233 (C:2a: P1.5) from serogroup C and strain H44/76 ( B:15:P1.7,16) from serogroup B. In all cases, the antisera protected infant rats against meningococcal infection.

### Example 8

### Generation of monoclonal antibody against protein NMB0928 able of mediating the bactericidal activity against Neisseria meningitidis

To generate monoclonal antibodies (mAbs) specific against protein NMB0928, and study the functional ability of mediating bactericidal activity against homologous and heterologous strain of *N. meningitidis,* an immunization schedule was conducted with a preparation of protein NMB0928 with purity higher than 70% (Example 3). The immunization was done in Balb/C (H-2^{d}, female, 5-6 weeks old) and 4 doses were applied as follows: On days 0, 15 and 30 of the immunization routine, 10 %g of antigen NMB0928 per mouse (total volume 100 µl), were administered by subcutaneous route, emulsified with Freund's Adjuvant; on day 50, 10 µg of antigen per mouse in Phosphate Buffered Saline (140 mM NaCl, 270 mM KCI, 1.5 mM KH₂PO₄, 6.5 mM Na₂HPO₄ x 2H₂O, pH 7.2) were administered by intra-peritoneal route. Blood extractions were done on days 0 and 45.

Splenocytes from the animal with the highest titer, measured by an indirect ELISA using protein NMB0928 as the coating antigen (Example 3), were fused with X63 Ag8 653 mouse myeloma cells. The resulting hybridomas were isolated and screened according to standard procedures (Gavilondo JV. 1995. Anticuerpos Monoclonales: Teoria y Práctica, Elfos Scientiae, La Habana, Cuba).

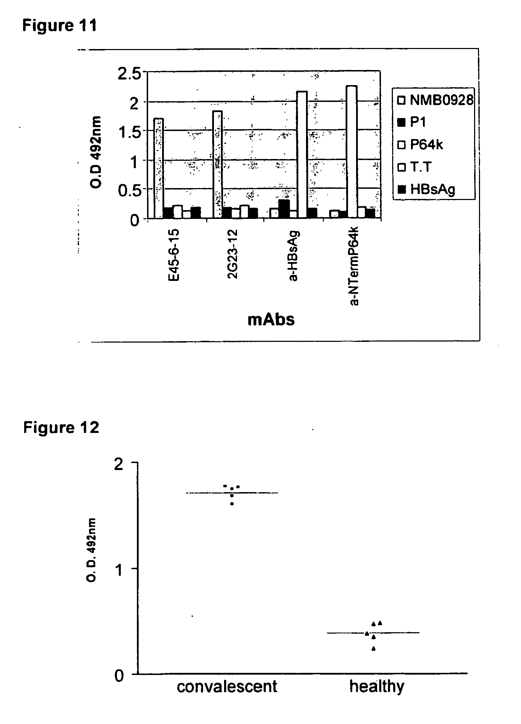

The reactivity of the antibodies secreted by the hybridomas directed to protein NMB0928, as well as their cross-reactivity non-related antigens, was tested by an indirect ELISA employing 5 µg/ml of each antigen, and the same concentration of each mAbs to be assayed. Figure 11 shows the results obtained in this experiment, all together 2 positive clones were obtained (mAbs E45-8-15 and 2G23-12) which specifically recognized protein NMB0928, and do not react neither with the amino acid sequence corresponding to the N-terminal of P64k, nor with the rest of the non-related antigens assayed.

To determine the ability of the mAbs generated against protein NMB0928 to mediate a bactericidal response against homologous and heterologous strains of *Neisseria meningitidis* a bactericidal test was performed. The bactericidal antibody titer was expressed as the reciprocal of the highest dilution of the antibodies tested that was able of killing 50% or more bacteria, the mAb 2G23-12 had bactericidal titers higher than 1:128 against the homologous strain B:4:P1.19,15 and higher than 1:64 against the heterologous strains B:15:P1.7,16 and C:2a:P1.5.

### Example 9

### Characterization of the target regions of the murine immune response against protein NMB0928

In order to identify the regions in the protein, which are more frequently recognized by the murine antisera generated against the recombinant antigen a SPOTScan assay was done. A set of overlapping peptides that span the sequence of the protein was synthesized on a cellulose membrane, which was incubated with pooled sera diluted 1:100. The antigen-antibody reaction was detected by the incubation with a conjugate anti-murine immunoglobulin G- alkaline phosphatase, followed by the addition of a solution that contained the substrate Bromo-chloro-indolyl-phosphate.

Several antigenic regions common within the protein were observed, no matter the preparation that was employed for the immunization. However, in the groups immunized with the protein adjuvated with Freund's Adjuvant there was a much broader pattern of recognition.

### Example 10

### Recognition of the NMB0928 protein by human sera.

A collection of human sera, coming from convalescent individuals was employed in this study, which was performed by ELISA. The plates were coated with protein NMB0928, obtained by preparative electrophoresis (5 µg/ml). Alter blocking the plates with 3% skim milk powder in PBS containing Tween-20, the sera were diluted (1:50) in the same solution and were incubated in the plates. The immunoassay continued as it has been widely reported. Healthy donor sera were employed as negative controls. In addition, pooled sera from individuals vaccinated with a recombinant vaccine against Hepatitis B was used a non-related control (data not shown).

Figure 12 shows the results obtained with 5 convalescent's sera in this assay. It can be seen that the human sera recognized the protein, which indicates that it is expressed during the meningococcal infection and it is immunogenic.

### Example 11

### Protein NMB0928 as a carrier for a peptide.

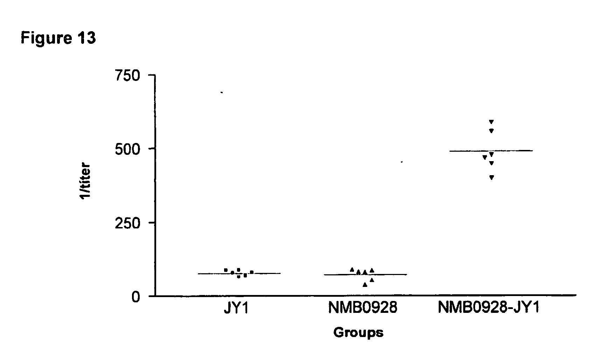

To demonstrate the carrier capacity of the recombinant protein NMB0928, it was conjugated to a 15 mer synthetic peptide, derived from the V3 region of protein gp120 from HIV-1, isolate JY1. The conjugation was done by the glutaraldehyde method. Free JY1 peptide, the recombinant protein NMB0928 and the conjugate JY1- NMB0928, were administered to adult mice in a 3-dose schedule, where the immunogens were emulsified with Freund's Adjuvant. Two weeks after the third dose, serum samples were obtained from the immunized animals, and the samples were analyzed by ELISA to determine the anti-peptide antibody titers. To do that, the plates were coated with free peptide (20µg/ml) and the immunoassay continued as it has been previously described. The results of the experiment (Figure 13) show the carrier capacity of protein NMB0928, able of significantly potentiate the antibody response against peptide JY1, after their conjugation.

## Claims

1. A composition comprising:
- the protein NMB0928 which consists of the amino acid sequence identified in the sequence listing as Seq. ID. No. 4, or
- a protein obtained by recombinant technology or chemical synthesis that comprises the amino acid sequence identified in the sequence listing as Seq. ID. No. 4,
for use as a medicament.

2. A composition according to claim 1, wherein said medicament is a vaccine.

3. A composition according to claim 2, wherein said vaccine generates a protective response in the recipient organism against infections caused by bacteria from the Neisseria genus.

4. A composition according to claim 3, wherein said vaccine generates a protective response in the recipient organism against infections caused by *Neisseria meningitidis.*

5. A composition according to claims 3 wherein said vaccine generates a protective response in the recipient organism against infections caused by *Neisseria gonorrhoea.*

6. Use of the protein NMB0928 which consists of the amino acid sequence identified in the sequence listing as Seq. ID. No. 4for the manufacturing a medicament for generating in the recipient organism a protective response against infections caused by bacteria from the Neisseria genus, more specific the infections caused by *Neisseria meningitidis* and *Neisseria gonorrhoea.*

7. Use of the protein identified in the sequence listing as Seq. ID. No. 4 as a carrier of antigens in the preparation of a carrier-antigen construct.

8. A composition **characterized by** comprising a carrier-antigen construct of the protein NMB0928 which consists of the amino acid sequence identified in the sequence listing as Seq. ID. No. 4 and at least one antigen, for use as a vaccine.

## Patentansprüche

1. Zusammensetzung umfassend:
- das Protein NMB0928, welches aus der in dem Sequenzprotokoll als Seq. ID. Nr. 4 angegebenen Aminosäuresequenz besteht, oder
- ein durch rekombinante Technologie oder chemische Synthese erhaltenes Protein, welches die in dem Sequenzprotokoll als Seq. ID. Nr. 4 angegebene Aminosäuresequenz umfasst,
zur Verwendung als ein Medikament.

2. Zusammensetzung nach Anspruch 1, wobei das Medikament ein Impfstoff ist.

3. Zusammensetzung nach Anspruch 2, wobei der Impfstoff in dem Empfängerorganismus eine schützende Antwort gegen Infektionen erzeugt, die durch Bakterien der Gattung Neisseria verursacht werden.

4. Zusammensetzung nach Anspruch 3, wobei der Impfstoff in dem Empfängerorganismus eine schützende Antwort gegen Infektionen erzeugt, die durch *Neisseria meningitidis* verursacht werden.

5. Zusammensetzung nach Anspruch 3, wobei der Impfstoff in dem Empfängerorganismus eine schützende Antwort gegen Infektionen erzeugt, die durch *Neisseria gonorrhoea* verursacht werden.

6. Verwendung des Proteins NMB0928, welches aus der in dem Sequenzprotokoll als Seq. ID. Nr. 4 angegebenen Aminosäuresequenz besteht, zur Herstellung eines Medikaments zum Erzeugen einer schützenden Antwort in dem Empfängerorganismus gegen Infektionen, die durch Bakterien aus der Gattung Neisseria verursacht werden, genauer gesagt die Infektionen, die durch *Neisseria meningitidis* und *Neisseria gonorrhoea* verursacht werden.

7. Verwendung des in dem Sequenzprotokoll als Seq. ID. Nr. 4 angegebenen Proteins als Träger von Antigenen bei der Herstellung eines Träger-Antigen-Konstrukts.

8. Zusammensetzung, **dadurch gekennzeichnet, dass** sie ein Träger-Antigen-Konstrukt aus dem Protein NMB0928, welches aus der in dem Sequenzprotokoll als Seq. ID. Nr. 4 angegebenen Aminosäuresequenz besteht, und wenigstens einem Antigen umfasst, zur Verwendung als ein Impfstoff.

## Revendications

1. Composition comprenant :
- la protéine NMB0928 qui consiste en la séquence d'aminoacides identifiée dans l'énumération de séquence en tant que Seq. ID. N° 4, ou
- une protéine obtenue par la technologie des recombinants ou par synthèse chimique, qui comprend la séquence d'aminoacides identifiée dans l'énumération de séquence en tant que Seq. ID. N° 4,
pour une utilisation comme médicament.

2. Composition selon la revendication 1, dans laquelle ledit médicament est un vaccin.

3. Composition selon la revendication 2, dans laquelle ledit vaccin produit une réponse protectrice dans l'organisme receveur, contre les infections provoquées par une bactérie du genre Neisseria.

4. Composition selon la revendication 3, dans laquelle ledit vaccin produit une réponse protectrice dans l'organisme receveur, contre les infections provoquées par *Neisseria meningitidis.*

5. Composition selon la revendication 3, dans laquelle ledit vaccin produit une réponse protectrice dans l'organisme receveur, contre les infections provoquées par *Neisseria gonorrhoea.*

6. Utilisation de la protéine NMB0928 qui consiste en la séquence d'aminoacides identifiée dans l'énumération de séquence en tant que Seq. ID. N° 4, pour la fabrication d'un médicament destiné à la production, dans l'organisme receveur, d'une réponse protectrice contre les infections provoquées par une bactérie du genre Neisseria, plus spécifiquement, les infections provoquées par *Neisseria meningitidis* et *Neisseria gonorrhoea.*

7. Utilisation de la protéine identifiée dans l'énumération de séquence en tant que Seq. ID. N° 4, comme véhicule pour antigènes dans la préparation d'une construction véhicule-antigène.

8. Composition **caractérisée en ce qu'**elle comprend une construction véhicule-antigène comprenant la protéine NMB0928 qui consiste en la séquence d'aminoacides identifiée dans l'énumération de séquence en tant que Seq. ID. N° 4 et au moins un antigène, pour une utilisation comme vaccin.
